# EUROPEAN PATENT APPLICATION

(11) **EP 2 138 180 A1**
(43) Date of publication of application: **30.12.2009**
(21) Application number: 09004026.2
(22) Date of filing: 20.03.2009
(51) Int. Cl.: A61K 33/00, A61K 41/00, A61P 35/00

(54) **A composition for treating cancer containing a porous silicon nanobomb as an active ingredient**

(30) Priority: 25.06.2008 KR 20080060110
(71) Applicant: INHA-INDUSTRY PARTNERSHIP INSTITUTE, Nam-ku Incheon 402-751 (KR)
(72) Inventor: Lee, Chongmu, Goyang-si Gyeonggi-do 410-761 (KR); Kim, Hohyeong, Cheonan-si Chungcheongsam-do 330-170 (KR); Hong, Chanseok, Incheon 402-873 (KR)
(74) Representative: Forstmeyer, Dietmar

(57) **Abstract**

Provided is a composition containing a porous silicon nanobomb as an active ingredient for treating cancer. The porous silicon nanobomb can be exploded by NIR light irradiation at a low intensity to selectively destroy cancer cells. Also, porous silicon itself shows good biocompatibility and biodegradability. Thus, the composition of the present invention can be used as an efficient anticancer agent without the accumulation of toxic side effects.

## Description

### BACKGROUND OF THE INVENTION

### 1. Field of the Invention

The present invention relates to a composition for treating cancer containing a porous silicon nanobomb capable of exploding upon near-infrared light irradiation. More particularly, the present invention relates to a composition for treating cancer containing a porous silicon nanobomb capable of selectively destroying cancer cells upon near-infrared light irradiation.

### 2. Description of the Related Art

Thermotherapy is a minimally invasive cancer treatment technique that can replace invasive surgical treatment. Entailing a relatively simple operation in addition to minimal invasiveness, thermotherapy makes possible a short recovery time. Also, thermotherapy is a type of physical therapy with fewer limitations than chemotherapy and is typically used in combination with both of the invasive therapies. In addition, it allows repeated treatments without the accumulation of toxic side effects. Thermotherapy (or thermal ablation therapy) includes laser-induced thermotherapy, microwave and radiofrequency (RF) ablation, magnetic thermal ablation, and focused ultrasound. Most of them, however, have shortcomings in that the treatment takes a long period of time and that its lesion boundaries are not well defined. Alternatively proposed was magnetic thermotherapy based on using alternating current to heat oxide nanoparticles in tumor cells. Although disadvantageous in that it requires a large quantity of iron for sufficient thermal effects, this magnetic thermotherapy has an advantage over the other thermotherapies in that it can selectively heat only the cells filled with oxides of iron.

In recent years, new thermotherapies based on a combination of nanoshells or single-wall carbon nanotubes (SWCNT) with near-infrared (NIR) light irradiation have attracted great attention because of their potent ability to kill cancer cells more selectively. These techniques are similar to photodynamic therapy (PDT) widely used in the clinic in that a drug (photosensitizing agent or thermo-coupling agent) is used in combination with light to cause selective damage to target cancer tissue. Both of these techniques use an extremely high amount of power to heat the thermal coupling agents, nanoshells and SWCNT to desired temperatures. For example, as high as 1 - 4 W/cm² is necessary for the near-infrared irradiation for SWCNT. Further, the heating of nanoshells needs 35 W/cm², which is too high to apply to the body (L. R. Hirch, J. Stafford R, J. A. Bankson, S. R. Sershen, B. Rivera, R. E. Price, J. D. Hazle, N. J. Halas, J. L. West, Proc. Natl. Acad. Sci. 2002, 100, 13549.). The heat of a light source used at high power in thermotherapy is likely to cause thermal damage to surrounding healthy cells even if they are not close to the thermal coupling agents such as nanoshells or SWCNT. Another research group has recently reported that the irradiation intensity of NIR light necessary to heat up SWCNT to a temperature high enough to kill cancer cells could be lowered down to the intensity level of the light commonly used in PDT by dispersing SWCNT in PBS solution prior to applying the SWCNT to cancer cells (T. Seki, M. Wakabayashi, T. Nakagawa, M. Imamura, T. Tamai, A. Nishimura, N. Yamashiki, A. Okamura & K. Inoue, Cancer (Philadelphia), 1999, 85, 1694.).

Disadvantages in the techniques taking advantage of the heat or explosion of carbon nanotubes with NIR irradiation in killing tumor cells (B. Panchapakesan, S. Lu, K. Sivakumar, K. Teker, G. Gesarone and E. Wickstrom, Nanobiotechnology, 2005, 1, 133. and N. W. S. Kam, M. O'connell, J. A. Wisdom and H. Dai, Proc. Natl. Acad. Sci. 2005, 102, 11600) have been found including the generation of reactive oxygen species toxic to the body and the toxicity of carbon nanotubes to the body.

Leading to the present invention, intensive and thorough research into cancer thermotherapy, conducted by the present inventors aiming to overcome the disadvantages encountered in the prior art, resulted in the finding that porous silicon, excellent in biocompatibility and biodegradability, can be heated as high and quickly as SWCNT during NIR light irradiation, thus making it appear more suitable for cancer therapy because of offering selectivity for cancer cells, and without toxicity and side effects.

### SUMMARY OF THE INVENTION

It is therefore an object of the present invention to provide a composition for treating cancer containing a biocompatible and biodegradable porous silicon nanobomb which can generate heat sufficient to selectively kill cancer cells upon exposure to NIR light at a low intensity, with accompaniment of neither toxicity to normal cells nor side effects.

In order to accomplish the above object, the present invention provides a composition for treating cancer containing a porous silicon nanobomb with excellent biocompatibility and biodegradability, prepared by mixing an oxidant with particles of a porous silicon layer formed on crystalline silicon through electrochemical etching.

### BREIF DESCRIPTION OF THE DRAWINGS

The above and other objects, features and other advantages of the present invention will be more clearly understood from the following detailed description taken in conjunction with the accompanying drawings, in which:
FIG. 1 is a schematic diagram of an electrochemical anodization cell for use in the preparation of nano-porous silicon;
FIG. 2 is an SEM showing nano-porous silicon in a plan view;
FIG. 3 is an SEM showing nano-porous silicon in a cross-sectional view;
FIG. 4 is an optical microphotograph showing a culture medium (DMEM) after exposure to NIR light at an intensity of 300 mW/cm² for 20 min;
FIG. 5 is an optical microphotograph showing a suspension prepared by suspending porous silicon particles with a size of 200 nm or less for 12 hrs in DMEM with agitation after exposure to NIR light at an intensity of 300 mW/cm² for 20 min;
FIG. 6 is an optical microphotograph showing a suspension prepared by suspending porous silicon particles with a size of 200 nm or less for 12 hrs in a 9% NaCl solution after exposure to NIR light at an intensity of 300 mW/cm² for 20 min;
FIG. 7 is an optical microphotograph showing a suspension prepared by suspending nano-porous silicon particles 200 nm or less in size with a sulfur(S) powder entrapped in the pores thereof for 12 hrs in a 9% NaCl solution with agitation after exposure to NIR light at an intensity of 300 mW/cm² for 20 min;
FIG. 8 shows the temperatures of the following samples upon exposure to NIR light at an intensity of 300 mW/cm² as a function of the NIR light irradiation time: a PSi/NaCl-suspension sample prepared by suspending the porous silicon particles of the Example for 12 hrs in a 9% NaCl solution with agitation; a porous silicon (PSi) layer sample formed on the surface of a monocrystalline silicon wafer; a PSi-suspension sample prepared by suspending the porous silicon particle in a culture medium; and a control sample of PSi treated with neither a suspension nor NIR;
FIG. 9 is a capture image of an explosion occurring upon the irradiation of NIR light onto the porous silicon bombs with NaClO₄·1H₂O entrapped within the pores thereof;
FIG. 10 is a capture image of an explosion occurring upon the irradiation of NIR light onto the porous silicon bombs with sulfur entrapped within the pores thereof;
FIG. 11 is optical microphotographs of breast cancer cells before NIR irradiation;
FIG. 12 is optical microphotographs of breast cancer cells after NIR irradiation for 20 min;
FIG. 13 is high-magnification microphotographs of breast cancer cells before NIR irradiation; and
FIG. 14 is high-magnification microphotographs of breast cancer cells after NIR irradiation at intensity of 300 mW/cm².

### DESCRIPTION OF THE PREFERRED EMBODIMENTS

In accordance with an aspect thereof, the present invention pertains to a composition for treating cancer containing a porous silicon nanobomb as an active ingredient which generates heat or is exploded by NIR light irradiation. When exposed to NIR light, the porous silicon nanobomb of the present invention heats as high and quickly as possible nanoshells or carbon nanotubes and is exploded to generate heat sufficient to kill cancer cells (Experimental Examples 2 and 4), with excellent biocompatibility and biodegradability, and accompanied by neither toxicity nor side effects.

The porous silicon nanobomb of the present invention can be heated by NIR light with an irradiation intensity of from 100 to 400 mW/cm², which is only about one hundredth of that required for heating nanoshells, that is, 35 W/cm². For carbon nanotubes, the irradiation intensity of NIR light amounts to 1 to 4 W/cm². Therefore, the present invention is useful in the treatment of cancer without damaging normal cells, in contrast to nanoshells and carbon nanotubes.

NIR wavelengths are within the range of from 0.78 to 1.4 µm. There are two important requirements for the light useful in the thermotherapy based on the porous silicon nanobombs: (1) it should be well absorbed by the porous silicon nanobombs; and (2) it should penetrate the human body well. Coincidence between the wavelengths of light for maximum absorption by porous silicon and for the highest transmittance to human tissues would make it ideal to use monochromatic radiation such as that of a laser using the optimum wavelength for thermotherapy because the highest efficiency of cancer cell destruction would be obtained by using it. Unfortunately, this is, however, not true in reality. The variation of the absorption coefficient of porous silicon with wavelength is similar to that of the absorption coefficient of crystalline silicon with wavelength, with the exception that the absorption coefficient of porous silicon is one or two orders smaller than that of crystalline silicon within a wavelength range of from 310 to 1200 nm. Porous silicon tends to gradually decrease in absorption coefficient with the shortening of light wavelengths from the infrared to visible spectral ranges.

On the other hand, biological systems including the human body are known to be highly transparent to 700 to 1,100 nm NIR light, with a maximum transmittance at near the wavelength of 808 nm. However, the transmittance does not change much with wavelength in this spectral range. When both the light absorption property of porous silicon and the penetration property of NIR light into human tissues are taken into consideration, thus, NIR light is appropriate for thermotherapy based on porous silicon, but does not necessarily have to be monochromatic. Heterochromatic radiation has an advantage over monochromatic radiation in that its source can be easily purchased at a much lower price than can a high power-laser diode for production of high-power monochromatic radiation. For the aforementioned reasons, heterochromatic radiation with a wavelength from 0.78 to 1.4 µm as well as monochromatic laser with a single wavelength in a range from 0.78 to 1.4 µm can be used.

The porous silicon nanobombs of the present invention is prepared by the following method, comprising:
electrochemically etching (anodization) crystalline silicon to form a porous silicon layer on a surface of the crystalline silicon (step 1); fracturing the porous silicon layer into porous silicon particles with a mean size of 220 nm or smaller (step 2); and mixing the porous silicon particles with an oxidant to allow the oxidant to infiltrate into pores of the porous silicon particles (step 3).

A detailed stepwise description is given of the preparation of porous silicon nanobombs, below.

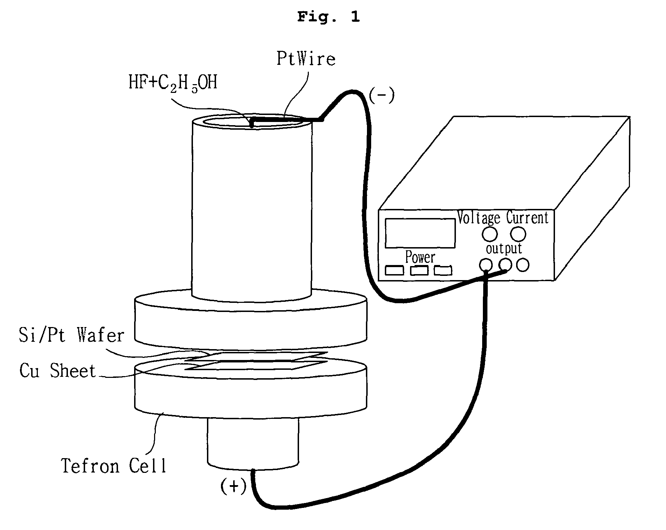

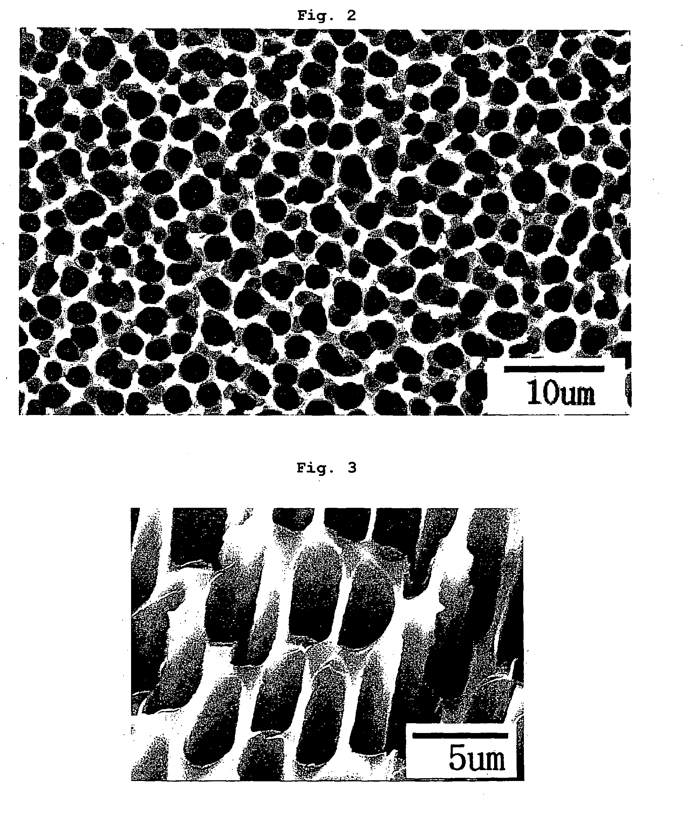

In step 1, crystalline silicon is made porous on the surface thereof. This can be done with electrochemical etching. In this case, the crystalline silicon pieces preferably range in specific resistance from 5 to 10 Ωcm. The crystalline silicon pieces in a mixture of 1:1 hydrogen fluoride (HF) : ethanol (C₂H₅OH) are electrochemically etched for 10∼30 min in the presence of a current density of from 20 to 70 mA/cm² to form a porous silicon layer on the surface of the crystalline silicon. Electrochemical cells used for the electrochemical etching (anodization) of silicon are shown in FIG. 1. Two types of pores are present in the porous silicon layer: cylindrical macropores with a diameter of ones of µm and a depth of tens of µm; and spherical micropores with a diameter of ones of nm (see FIG. 2 and 3).

In step 2, porous silicon particles are obtained by fracturing the porous silicon layer formed in step 1. Step 2 can be conducted with an ultrasonicator. In greater detail, the crystalline silicon pieces with a porous silicon layer formed thereon are subjected to ultrasonication in water to give off porous silicon as nano-particles, followed by filtration through a 220 nm membrane to obtain porous silicon particles with a size of 220 nm or smaller. Therefore, these porous silicon particles have nano-pores, but no macropores are found therein.

Step 3 is to mix the porous silicon particles obtained in step 2 with an oxidant to afford the porous silicon nanobombs of the present invention. Examples of the oxidant useful in step 3 include sulfur, Gd (NO₃)₃·6H₂O, NaClO₄·1H₂O etc. Among them, sulfur is more preferred because of its relative unreactiveness to normal cells compared with Gd(NO₃)₃·6H₂O or NaClO₄·1H₂O. In greater detail, the porous silicon particles obtained in step 2 are dipped in a solution of an oxidant powder, such as (NH₄)₂S solution for sulfur, etc., so that the oxidant infiltrates into the nanopores of the porous silicon particles. Then, the porous silicon particles are dried to remove moisture in the pores.

In an embodiment of the present invention, the composition for treating cancer is suspended together with folic acid in saline and the suspension is intravenously administered to patients suffering from cancer. The silicon nanoboms thus coated with folic acid move to cancer cells. After being internalized into the cancer cells, the porous silicon nanobombs are exploded by NIR irradiation to blow up the tumor. Data obtained in an experiment with the anticancer agent comprising the porous silicon nanobombs according to the present invention (Experimental Example 1), as shown in Table 1, gives a cancer cell viability of 3.7 % or less upon the use of the porous silicon nanobombs in combination with NIR radiation (E and F), indicating that the anticancer agent of the present invention is efficient in the treatment of cancer.

In accordance with a still further aspect thereof, the present invention pertains to a use of the above porous silicon nanobomb a manufacture of a medicament for treating cancer.

A better understanding of the present invention may be obtained through the following examples which are set forth to illustrate, but are not to be construed as limiting the present invention.

### PREPARATION EXAMPLE: Preparation of Porous Silicon NanoBombs

On a crystalline silicon piece with dimensions of 2.5 cmx2.5 cm×0.05 cm and a specific resistance of about 10 Ωcm, electrochemical etching was conducted for 10 min at a current density of 50 mA/cm² in a mixture of 1:1 HF : ethanol (C₂H₅OH). The porous silicon layer thus obtained was found to be 73% in porosity and 55 µm in thickness as measured by weight measurements. In the porous silicon layer were observed cylindrical macropores and spherical micropores (see FIG. 2 and 3).

Subsequently, the porous silicon layer formed on the crystalline silicon piece was fractured into particles by ultrasonication in water, followed by filtration through a 220 -nm membrane to afford porous silicon particles with a size of 220 nm or less.

Then, the porous silicon particles were dipped in 45 wt.% (NH₄)₂S solution for 10 min so that the sulfur was allowed to infiltrate into the micropores of the silicon particles. Next, the porous silicon particles were dried by a nitrogen gun first and then in an oven at 50°C for 2 hrs. As a result, porous silicon nanobombs with sulfur captured in the pores thereof were obtained.

### EXPERIMENTAL EXAMPLE 1: Therapeutic Effect of the cancer therapy Based on Porous Silicon Nanobomb

The porous silicon nanobomb prepared in the Preparation Example were dispersed in a 9% NaCl solution with agitation to give a PSi/NaCl/S suspension. Separately, breast cancer SK-BR-3 cells were cultured in DMEM (Dulbeco's Modified Eagle's Medium). SK-BR-3 cells were seeded at a density of 1×10⁵ cells per well onto 24-well plates for 18 hrs and then incubated at 37°C for 24 hrs under a 5% CO₂ atmosphere. Then, the culture medium was aspirated and the cancer cells were washed with PBS before the addition of fresh DMEM to each well. The breast cancer cell samples thus prepared were exposed to NIR light under an NIR lamp (Model IF-9900 Gold, Hasell CO., USA, wavelength = 0.718 - 1.4 µm, irradiation intensity = 300 mW/cm²) in the following six patterns, and the results are given in Table 1 and FIGS. 3-7, 11 and 12.
A. control cells (treated with neither PSi nor NIR).
B. cells treated with PSi/NaCl suspension
C. cells treated with PSi/S/NaCl suspension.
D. cells exposed to NIR light for 20 min in the absence of PSi.
E. cells exposed to NIR light for 20 min in the presence of PSi/NaCl suspension.
F. cells exposed to NIR light for 20 min in the presence of PSi/S/NaCl suspension.

**TABLE 1**

| sample | Treatment | Cell viability (%) | | | |
|---|---|---|---|---|---|
| | | Test 1 | Test 2 | Test 3 | Avg. |
| A | None | 104.4 | 97.6 | 103.1 | 101.7 |
| B | PSi/NaCl | 96.3 | 100.8 | 98.7 | 98.6 |
| C | PSi/S/NaCl | 96.4 | 98.3 | 97.6 | 97.4 |
| D | NIR | 95.2 | 98.4 | 94.7 | 96.1 |
| E | PSi/NaCl + NIR | 5.4 | 4.5 | 1.2 | 3.7 |
| F | PSi/S/NaCl + NIR | 3.8 | 2.7 | 2.4 | 3.0 |

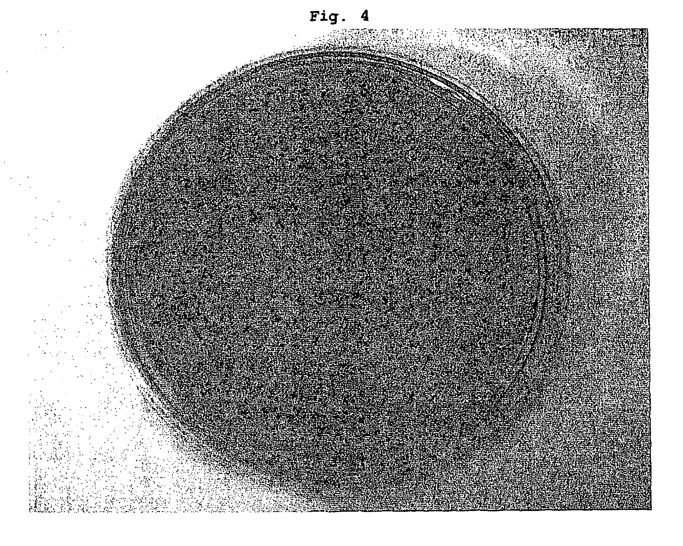

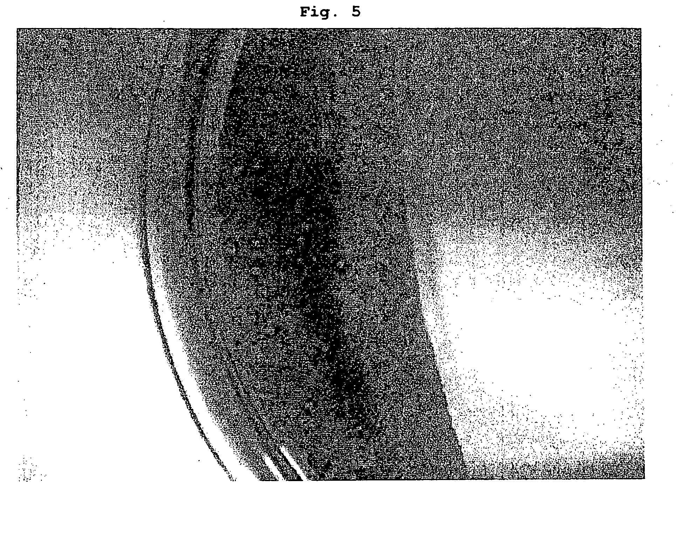

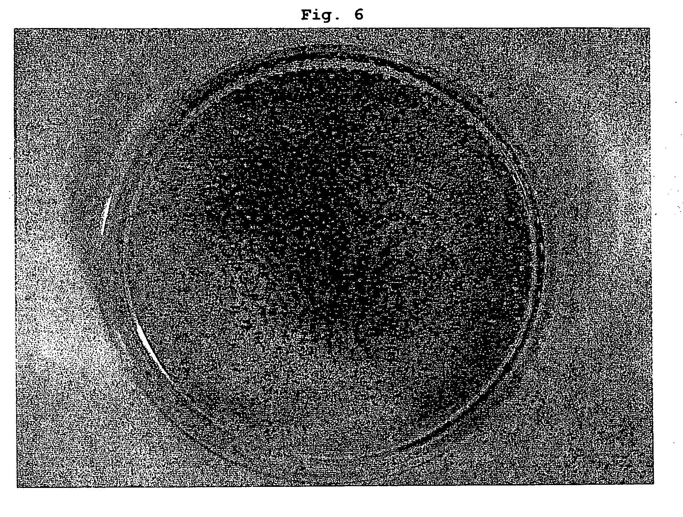

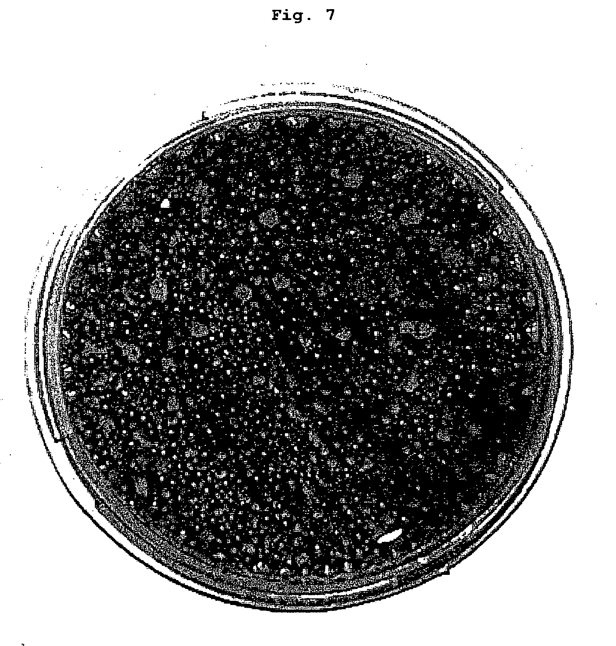

As seen in Table 1, the cells were near 100% alive (respectively 101.7, 98.6 and 97.4%) after treatment (A) with neither NIR nor PSi (FIG. 4), (B) with PSi/NaCl alone or (C) with PSi/S/NaCl alone (FIG. 5). Further, NIR alone could not decrease the viability of cancer cells (96.1 %), indicating that PSi and NIR light cannot kill cells when used alone. In contrast, the viability was greatly decreased to 3.7% and 3.0% respectively when NIR light was used in combination with a PSi/NaCl suspension (E)(FIG. 6) and a PSi/S/NaCl suspension (F)(FIG. 7). These results demonstrate that when exposed to NIR light, the porous silicon nanobombs according to the present invention are very effective in killing cancer cells.

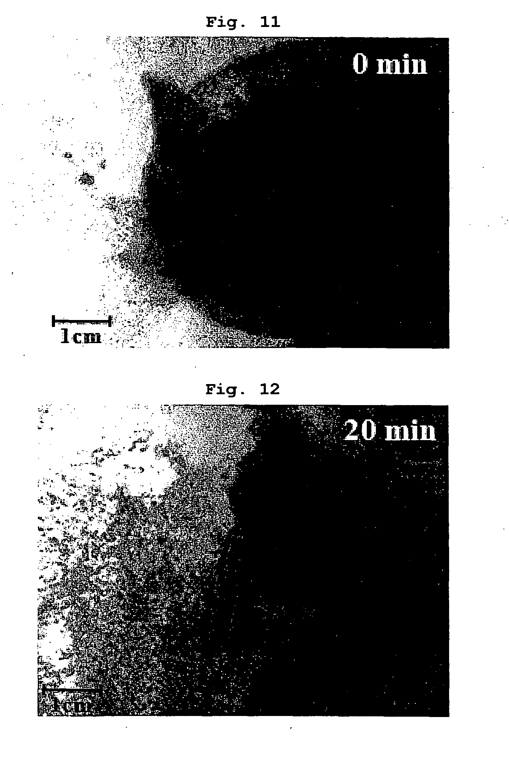

FIG. 11 is microphotographs of PSi/S/NaCl-treated breast cancer cells before exposure to NIR light, and FIG. 12 is that of after exposure to NIR light for 20 min. A drama is seen between the cells of FIGS. 12 and 11. Upon NIR exposure in the presence of the PSi suspension, the cells seemed to be blown up and burnt black. Explosion was observed to occur inside the cell clusters as inferred from the morphology of dead cells. Also, the bubbles showed that the cells were blown up in the explosion. Bubbles found around dead cells were evidence of the vigorous boiling of the NaCl solution within porous silicon particles, implying that the explosion of porous silicon particles resulted from the temperature elevation of the NaCl solution localized within the silicon particles to exceed the boiling point.

### EXPERIMENTAL EXAMPLE 2: Heat Emission of Porous Silicon Nanobomb upon NIR Irradiation at Low Intensity

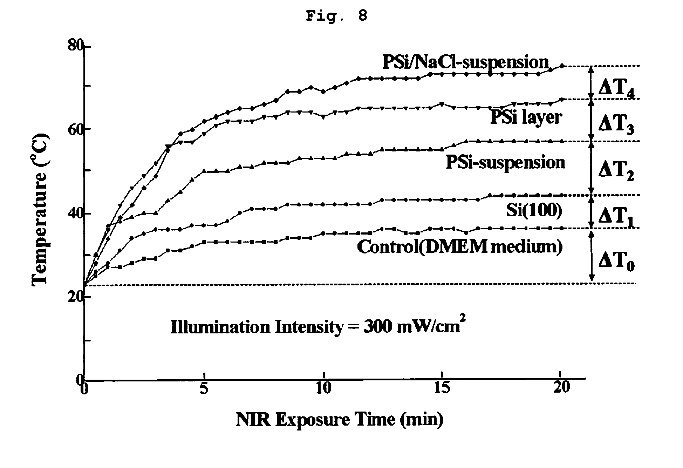

When exposed to NIR light at 300 mW/cm² for 20 min, the four samples were measured for temperature: a PSi/NaCl-suspension sample prepared by suspending the porous silicon particles of Preparation Example for 12 hrs in a 9% NaCl solution with agitation; a porous silicon (PSi) layer sample formed on the surface of a monocrystalline silicon wafer; a PSi-suspension sample prepared by suspending the porous silicon particle in a culture medium; and a control sample being PSi treated with neither suspension nor NIR. The results are depicted in FIG. 8.

All of the samples, as shown in FIG. 8, increased in temperature in parabolic patterns with the NIR exposure time. The PSi/NaCl-suspension sample was heated to 55°C after 3 min exposure and 74°C after 15 min exposure while the temperature of the control sample was elevated only to 31°C and 39°C after 3 min and 20 min, respectively. The temperature difference (AT = ΔT₁ + ΔT₂ + ΔT₃ + ΔT₄) after 20 min NIR irradiation between the PSi/NaCl-suspension sample and the control sample was 37°C, which corresponds to the net heating effect of the PSi/NaCl-suspension. This temperature difference (ΔT = 37°C) is almost the same as that (ΔT = 37.4°C) obtained by Hirsch et al. using nanoshells in combination with NIR light at a density of 35 W/cm². This supports the important connotation that substitution of nanoshells with PSi can substantially lower the irradiation intensity of NIR necessary to obtain a heating effect sufficient to destroy cancer cells down to a level (300 mW/cm²) which can be actually used in the clinic.

Comparison of the three different temperature curves for PSi/NaCl-suspension, PSi-suspension and control samples shows that the net heating effects of PSi (ΔT_{PSi} = ΔT₁ + ΔT₂) and NaCl solution (ΔT_{NaCl} = ΔT₃ + ΔT₄) are 18°C and 19°C, respectively, after 20 min NIR irradiation and 9°C and 13°C, respectively, after 3 min NIR irradiation. The difference in the temperature of the control sample after NIR irradiation for 0 and 20 min (ΔT₀) is 16°C, which is attributable mostly to the heat emitted from the NIR light source at an irradiation intensity of 300 mW/cm² because the net heating effect of the control sample itself due to absorption of NIR by DMEM is thought to be negligible.

### EXPERIMENTAL EXAMPLE 3: Explosivity of Porous Silicon Nanobomb

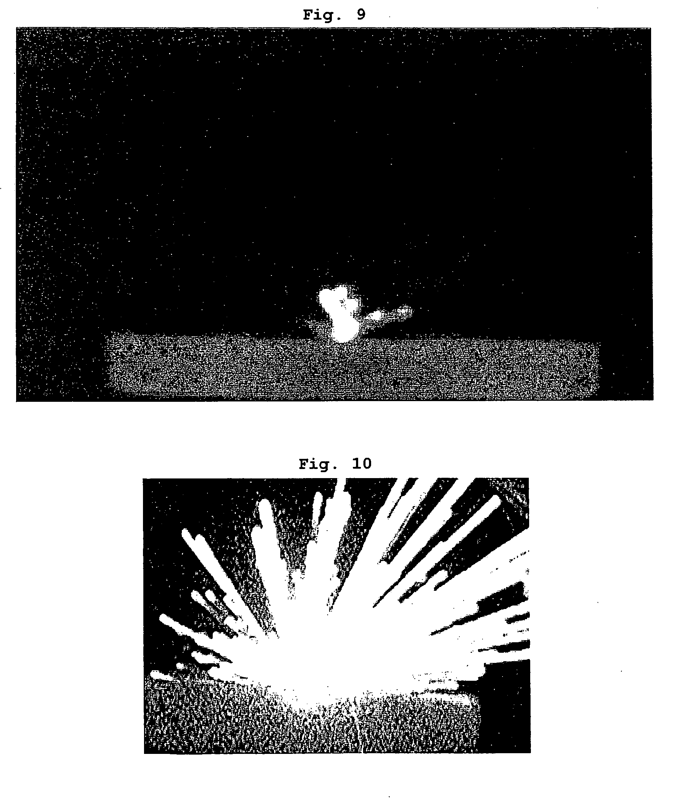

Porous silicon nanobomb samples were prepared in the same manner as in the Preparation Example, with the exception that NaClO₄·1H₂O was used as an oxidant instead of sulfur. These porous silicon nanobomb samples were placed in respective plates and exposed to NIR light at an irradiation intensity of 300 mW/cm². The subsequent explosions were captured and are shown in FIGS. 9 and 10.

As seen in FIGS. 9 and 10, the bombs of sulfur (FIG. 10) were exploded on a larger scale than were the bombs of NaClO₄·1H₂O (FIG. 9).

### EXPERIMENTAL EXAMPLE 4: Selectivity of Porous Silicon Nanobombs for Cancer Cells

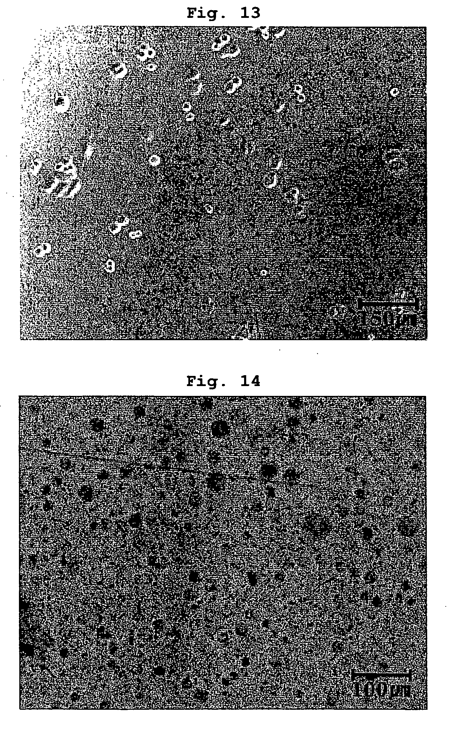

The PSi/S/NaCl suspension prepared by suspending the porous silicon nanobombs of the Preparation Example in a 9% NaCl solution was applied to a central region of a cluster of breast cancer cells (SK-BR3) on a plate. Upon NIR exposure at an irradiation intensity of 300 mW/cm², the cells turned blue as a result of the staining of cell membranes with Trypan blue. FIGS. 13 and 14 shows cells before and after NIR irradiation, respectively. Only the central region to which the PSi/S/NaCl suspension was applied turned blue, indicating that the porous silicon nanobombs of the present invention selectively destroy cancer cells.

As described hitherto, the porous silicon nanobombs according to the present invention can be exploded by NIR light irradiation at a low intensity and are highly selective for cancer cells. Thus, the porous silicon nanobombs can selectively destroy cancer cells in a repeated manner without the accumulation of toxic side effects.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A composition for treating cancer containing a porous silicon nanobomb as an active ingredient, functioning in such a manner that the porous silicon nanobomb concentrates at a tumor locus of cancer cells, and then emits heat or is exploded by near-infrared irradiation to remove the cancer cells.

2. The composition according to claim 1, wherein the near-infrared light ranges in irradiation intensity from 100 to 400 mW/cm².

3. The composition according to claim 1, wherein the near-infrared light is heterochromatic light with a wavelength range from 0.78 to 1.4 µm or monochromatic laser with a single wavelength in a range from 0.78 to 1.4 µm.

4. The composition according to claim 1, wherein the porous silicon nanobomb is prepared by the following method, comprising:
electrochemically etching crystalline silicon to form a porous silicon layer on a surface of the crystalline silicon (step 1);
fracturing the porous silicon layer into porous silicon particles with a mean size of 220 nm or smaller (step 2); and
mixing the porous silicon particles with an oxidant to allow the oxidant to infiltrate into pores of the porous silicon particles (step 3).

5. The composition according to claim 4, wherein the electrochemical etching of step 1 is conducted in a mixture of 1:1 hydrogen fluoride (HF) : ethanol (C₂H₅OH).

6. The composition according to claim 4, wherein the porous silicon layer of step 1 has cylindrical macropores and spherical microphores therein.

7. The composition according to claim 4, wherein the fracturing of step 2 is carried out using an ultrasonicator in water.

8. The composition according to claim 4, wherein the porous silicon particle of step 2 contains only pores which are nanometers in size.

9. The composition according to claim 4, wherein the oxidant is sulfur, and is infiltrated into the pores of porous silicon by dipping the porous silicon in (NH₄)₂S solution.

10. A Use of the porous silicon nanobomb of claim 1 in a manufacture of a medicament for treating cancer.
